# EUROPEAN PATENT APPLICATION

(11) **EP 1 375 671 A1**
(43) Date of publication of application: **02.01.2004**
(21) Application number: 02077575.5
(22) Date of filing: 28.06.2002
(51) Int. Cl.: C12P 1/00, C12P 3/00, C12P 7/02, C12P 7/38, C12P 7/62, C12P 11/00, C12P 17/02, C12P 19/32, C12P 19/36, C12M 1/40, C12N 9/02, C12N 11/08, C01B 13/02, C07H 19/207

(54) **Selective functionalization of hydrocarbons with isolated oxygenases and mediator-based regeneration**

(71) Applicant: Eidgenössische Technische Hochschule Zürich, 8093 Zürich (CH)
(72) Inventor: Schmid, Andreas, 8049 Zürich (CH); Hollmann, Frank, 8046 Zürich (CH); Witholt, Bernard, 8032 Zürich (CH)
(74) Representative: Prins, Adrianus Willem

(57) **Abstract**

Methods for preparative *in vitro* biocatalysis utilizing oxidoreductases. These methods are based on approaches to supply the oxidoreductases with reduction equivalents derived either from chemical or electrochemical sources. By utilization of organometallic mediators, the oxidoreductases are regenerated directly in the absence of nicotinamide coenzymes and biocatalysts generally used to transfer the reduction equivalents to the active site.

These methods also include reactor design for continuous electrochemical regeneration of these enzymes.

## Description

### Background of the Invention

Oxofunctionalized hydrocarbons are important synthons e.g. for the synthesis of various pharmaceutically relevant compounds. This significance has driven the research for practical routes to obtain preferably enantiopure oxygenated products such as alcohols, lactones, phenols and epoxides from simple starting materials in high yields. Most prominent chemical procedures are the Sharpless epoxidation of allylic alcohols and the method of Jacobsen and Katsuki. In addition to these methods biomimetic epoxidation procedures have gained a great deal of attention in recent years. Despite tremendous advances here, these chemical approaches seldom meet the catalytic performances of biocatalytic reactions in terms of rate enhancement, reaction conditions, substrate tolerance, and regio-, chemo- and stereoselectivity.

A couple of enzyme classes are known to catalyze epoxidation reactions and oxygenation reactions in general. Amongst these peroxidases and monooxygenases have gained particular attention and will be described in more detail in the following sections.

### Peroxidases

Peroxidases utilize hydrogen peroxide as activated oxygen to perform oxygenation reactions. Due to the high oxidation potential of the resulting enzyme-oxo-species a broad variety of oxygenation reactions such as epoxidation, hydroxylation, halogenation, hetero-atom oxidation and oxidations of unactivated C-H bonds have been described [1].

However, peroxidases depend on stochiometric amounts of hydrogen peroxide exhibits a destructive impact on enzymatic activity thus necessitating *in situ* control of H₂O₂-concentration. Approaches such as use of H₂O₂ precursors like *tert.*-butyl peroxide [2], controlled dosing of H₂O₂ (feed-on-demand strategies) [2], *in situ* generation of H₂O₂ either electrochemically [3] or by means of an oxidase reaction [2] have been reported.

### Monooxygenases

### Approaches for NAD(P)H regeneration

Most monooxygenases indirectly depend on reduced nicotinamide coenzymes (NAD(P)H) whose reduction equivalents are transferred to the monooxygenase in most cases via a reductase. There are only few examples of monooxygenases being capable of utilizing NAD(P)H directly without additional reductases. Examples are aromatic hydroxylases such as 2-hydroxybiphenyl-3-monooxygenase (HbpA, E.C. 1.14.13.44), toluene-4-monooxygenase or cyclohexanone monooxygenase (CHMO, Baeyer-Villiger-monooxygenase, E.C. 1.14.13.22).

Established approaches for *in vitro* regeneration of these monooxygenases usually comprise regeneration of the costly NAD(P)H cofactor. Schmid et al. for example used formate dehydrogenase (FDH, E.C. 1.2.1.2) to regenerate NADH in a 2-liquid phase system for the *ortho*-hydroxylation of 2 hydroxybiphenyl with the enzyme 2-hydroxybiphenyl 3-monooxygenase (HbpA, E.C. 1.14.13.44) [4]. Utilizing an engineered FDH Rissom et al. performed *in situ* regeneration of NADPH for a cyclohexanone monooxygenase-catalyzed (CHMO, E.C. 1.14.13.22) oxidation of 4-methyl cyclohexanone to (S)-5-methyl-oxepane-2-one [5].

NAD(P)H was also regenerated utilizing alcohol dehydrogenases such as the alcohol dehydrogenase from *Thermoanaerobium brokii* (TBADH, E.C. 1.1.1.1) or other sources.

Willets et al. used a coupled enzymatic approach of dehydrogenase and Baeyer-Villiger monooxygenase from *Acinetobacter calcoaceticus* NCIMB 9871 and *Pseudomonas putida* NCIMB 10007 to transform alcohols via the keton into lactones [6-8]. The resulting bienzymatic systems however are seldom easily optimizable due to varying demands of both enzymes with respect to reaction conditions such as temperature, buffer, pH, substrate- and product tolerance, etc.

Other enzymatic procedures for the regeneration of NADPH have been reported comprising glucose-6-phosphate dehydrogenase [9], ferredoxin-NADP reductase [10] and various others. Other approaches mainly comprise electrochemical methods utilizing viologene derivates [11] acting on a NAD(P)⁺ reductase. Due to the high instability toward molecular oxygen these approaches are so far limited to bioconversions in oxygen-free media. [Cp*Rh(bpy)(H₂O)]²⁺ has been described as efficient catalyst for the reduction of NAD(P)⁺ into the enzyme-active reduced form [12]. It was applied either under chemical [13] or electrochemical regeneration [14] to regenerate NADH in dehydrogenase-catalyzed reduction reactions. Its applicability to NADH regeneration coupled to monooxygenase reactions was shown recently by performing chemically or electrochemically driven regeneration of NADH combined to a HbpA catalyzed hydroxylation reaction [15] [16].

### Approaches for direct regeneration of oxidoreductases

As mentioned above, monooxygenases need reduction equivalents to activate molecular oxygen. Mostly these reduction equivalents are derived from reduced nicotinamide coenzymes. However, in most cases the active (monooygenating) site is not directly regenerated by NAD(P)H but rather via a more or less complicated electron transport chain. Direct regeneration in this context means shortage of this electron transport chain and ideally transferring the reduction equivalents exclusively to the active site thus eliminating NAD(P)H and the reductase and mediator.

Vilker et al reported the electrochemical regeneration of cytochrome P450_{cam} monooxygenases via cathodic reduction of putidaredoxin, eliminating NADH and putidaredoxine reductase from the regeneration cycle for the monooxygenase. Thus electro-enzymatic hydroxylation of camphor [17] and epoxidation of styrene [18] were achieved.

Inorganic mediators have also been used to regenerate P450 monooxygenases. For example Schmid and coworkers used Co-sepulchrate as electron mediator between elemental zinc and P450 BM-3 mutants (fusion protein of monooxygenase and reductase component) to perform ω-hydroxylation of fatty acid derivatives [19]. Estabrok and coworkers have shown electrochemical reduction of Co-sepulchrate to transfer electrons to a recombinant rat liver P450 fusion protein to convert lauric acid [20].

Direct electroreduction for P450cam was reported [21] as well as for Cytochrome C (sharing structural and electronic similarities with the P450 monooxygenases) [42]. However, only the occurrence of heterogeneous electron transfer between the electrode surface and the heme group was reported.

Nolte et al. used [Cp*Rh(bpy)L]-complexes as catalysts for the reduction of Mn-porphyrin complexes [22] and the non-stereoselective epoxidation of styrene, cis-stilbene, α-pinene and nerol. However, no reduction of enzyme-bound heme was shown.

Arnold et al used hydrogen peroxide to regenerate P450 monooxygenases. This so-called hydrogen peroxide shunt was used as coenzyme-free regeneration system for P450 cam [23]. Here, hydrogen peroxide was used instead of O₂ and NADH to drive a P450-catalyzed hydroxylation of naphthalene.

For cytochrome c, a P450-like activity was reported [24], for example sulfoxidation of thioanisol. Here, again the hydrogen peroxide shunt was used to drive the enzymatic reaction.

Probably due to the destructive impact of hydrogen peroxide on enzymatic activity, no preparative application of the hydrogen peroxide shunt was reported so far.

### Dehydrogenases

### Approaches for the regeneration of NAD(P)⁺ from the reduced form

Oxidation reactions can also be catalyzed by dehydrogenases. In the course of this reaction, generally NAD(P)H is produced from NAD(P)⁺, necessitating regeneration of the costly oxidized cofactor. General methods for this task have been reviewed recently comprising mainly enzymatic procedures [2].

Enzyme-free approaches such as direct electrochemical oxidation of NAD(P)H [25] or mediated electrochemical oxidation [26] have been reported.

Utilizing molecular oxygen as terminal acceptor has been suggested. Reduced alloxazine moieties can oxidize NAD(P)H while being reoxidized by molecular oxygen. The product of this reaction, hydrogen peroxide can be dismutated by catalase. However, non-catalyzed transhydrogenation between NAD(P)H and alloxazine (e.g. FAD, FMN) is very slow and can be enhanced by enzymatic catalysis [27].

We have reported, that [Cp*Rh(bpy)(H₂O)]²⁺ can act on NAD(P)H leading to a species not absorbing at 340 nm. However, it was not shown that the product of this reaction is enzyme active NAD(P)⁺. Furthermore, Koelle and Ryabov reported insufficient selectivity of related [(Cp*Rh)₂ (□-Cl)₃]⁺ complexes [28].

### Summary of the invention

The present invention relates to the biocatalytic synthesis of chemical compounds, in particular the *in vitro* application of coenzyme dependant oxidoreductases and methods to regenerate the enzymes.

In particular, the invention relates to the application of organometallic compounds to catalyze the regeneration of the oxidoreductases directly. This includes drastic simplification of the electron transfer chain from the stochiometric source of reduction equivalents (chemical compound or cathode) to the active site of the oxidoreductase leaving out costly and instable coenzymes such as NAD(P)H and enzymatic catalyst such as reductases and electron transfer proteins such as putidaredoxin.

The present invention concerns the biocatalytic production of specifically oxofunctionalized hydrocarbons.

This includes a novel method of providing cell-free biocatalyst with reduction equivalents needed for oxofuctionalization and experimental setups for this reaction.

In particular, the invention relates to the specific coupling of organometallic complexes like [Cp*Rh(bpy)(H₂O)]²⁺ as electron transfer reagent to functional enzyme parts for selective epoxidations, sulfoxidations, Baeyer-Villiger oxidations and reduction of oxygen itself.

### Brief description of the drawings

Figure 1: In vitro regeneration of styrene monooxygenase (StyA). [Cp*Rh(bpy)(H₂O)]²⁺ catalyzed regeneration (upper) compared to a reductase-catalyzed setup (e.g. utilizing the native reductase StyB) with NAD(P)H regeneration.
Figure 2: Examples for StyA-cataylyzed oxidation reactions.
Figure 3: [Cp*Rh(bpy)H]⁺ catalyzed and formate driven reduction of CytC. Electrons are derived either from chemical reductants (such as formate) or from the cathode.
Figure 4: Summarized regeneration pathways of in vitro regeneration of monooxygenases and peroxidases.
Figure 5: Transhydrogenation from NAD(P)H to FAD (FMN) catalyzed by Cp*Rh(bpy)(H₂O)]²⁺ and its application to dehydrogenase catalyzed oxidation reactions.
Figure 6: Schematic setup of a compartmented electrochemical setup with immobilized biocatalyst. (1) stirred reservoir for substrates and products in a suitable solvent; (2) pump; (3) hollow-fibre module; (4) flow-through electrolysis cell (connected to a potentiostat); (5) pump; (6) immobilized biocatalyst (7) thermostat (8) pump.
Figure 7: UV-spectra of CytC while incubation with hydrogen peroxide.
Figure 8: Experiments on varying c([Cp*Rh(bpy)(H₂O)]²⁺) and c(CytC).
Figure 9: Sub-stoichiometric use of [Cp*Rh(bpy)(H₂O)]²⁺. c([Cp*Rh(bpy)(H₂O)]²⁺) = 10 µM, c(cytC)= 80 µM, c(NaHCO₂) = 150 mM, T= 30°C, degassed buffer.
Figure 10: Time course of [Cp*Rh(bpy)(H₂O)]²⁺-driven and StyA-catalyzed epoxidation of styrene. Styrene oxide (solid diamonds); styrene (solid squares). T = 37°C; c(NaHCO₂) = 0.15 M; c([Cp*Rh(bpy)(H₂O)]²⁺) = 2 x 10⁻⁴ M: c(FAD) = 5 x 10⁻⁵ M; c(FMN) = 5 x 10⁻⁶ M: catalase = 28 U x ml⁻¹; cₒ (styrene) = 2 x 10⁻³ M; c(StyA) = 50 µg x ml⁻¹ = 1.16 x 10⁻⁶ M
Figure 11: Styrene oxide formation in the presence of neat styrene as 2nd organic phase (substrate & product reservoir).
Figure 12: Styrene oxide concentration after 15 min incubation on variation of c(StyA).
Figure 13: Styrene oxide concentration after 15 min incubation on variation of c([Cp*Rh(bpy)(H₂O)]²⁺).
Figure 14: Styrene oxide formation rate as a function of c(FAD). c([Cp*Rh(bpy)(H₂O)]²⁺) = 0.2 mM; c(StyA) = 1.25 µM; Catalase 280 U; c(styrene) = 2 mM; T = 37°C.
Figure 15: Styrene oxide (StyOx) formation using immobilized StyA.
Figure 16: Time course of dissolved oxygen (DOT) (solid circle) and c(FADH₂) (open circle) while incubating with [Cp*Rh(bpy)(H₂O)]²⁺ (0.2 mM) in sodium formate (0.15 M) at 37°C.
Figure 17: Time course of hydrogen peroxide formation at different ratios [Cp*Rh(bpy)(H₂O)]²⁺ / FAD. c([Cp*Rh(bpy)(H₂O)]²⁺) = 19 µM, c(NaHCO₂) = 0.15 M, T = 37°C, c(FAD) = 0 µM (solid circle), 10 µM (open circle), 20 µM (solid diamond), 50 µM (open diamond), 100 µM (solid triangle), 200 µM (open triangle).
Figure 18: UV-spectra of a 50 µM Cyt C solution in the presence of 1 mM H₂O₂.
Figure 19: Dependence of the CytC-catalyzed sulfoxidation efficiency on c(CytC).
Figure 20: Time-course of CytC-catalyzed sulfoxidation of thioanisol with in situ generation of hydrogen peroxide by [Cp*Rh(bpy)(H₂O)]²⁺.
Figure 21: Residual HbpA activity while incubation with [Cp*Rh(bpy)(H₂O)]²⁺ and varying NH₃ concentrations.
Figure 22: Inhibition of formate driven NADH regeneration catalyzed by [Cp*Rh(bpy)(H₂O)]²⁺ under varying NH₄⁺ concentrations.
Figure 23: Feasibility of electrochemical NADH regeneration in NH₄⁺ containing buffers.

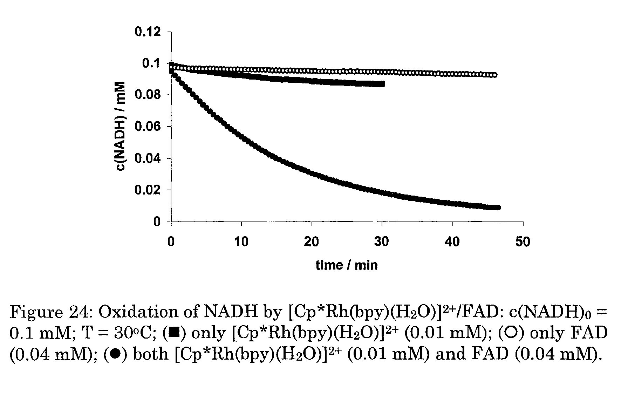

Figure 24: Oxidation of NADH by [Cp*Rh(bpy)(H₂O)]²⁺/FAD: c(NADH)₀ = 0.1 mM; T = 30°C; (solid square) only [Cp*Rh(bpy)(H₂O)]²⁺ (0.01 mM); (open circle) only FAD (0.04 mM); (solid circle) both [Cp*Rh(bpy)(H₂O)]²⁺ (0.01 mM) and FAD (0.04 mM).
Figure 25: Time course of the oxidation of 3-methyl cylohexanol catalyzed by alcohol dehydrogenase from *Thermus sp..* The necessary oxidized nicotinamide coenzyme was in situ generated from NADH.

### List of Tables

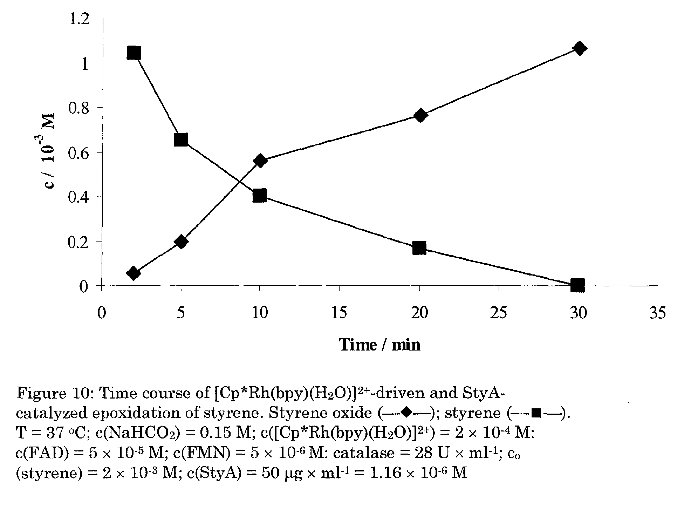

Table 1: CytC reduction rates at varying temperatures. (c(CytC) =31.2 µM, c(NaHCO₂) = 150 mM).
Table 2: Typical reaction conditions.
Table 3: Characteristics for the chemo-enzymatic epoxidation reaction represented in Figure 10.
Table 4: Substrates. (Reaction conditions: T = 37°C, c([Cp*Rh(bpy)(H₂O)]²⁺) = 0.2 mM, c(StyA) = 1.47 µM, c(NaHCO₂) = 150 mM, c(FAD) = 20 µM, c(Catalase) = 200 U*ml⁻¹.
Table 5: CytC inactivation as a function of [CytC] and [H₂O₂].
Table 6: Residual activities of HbpA while incubation with [Cp*Rh(bpy)(H₂O)]²⁺ .

### Detailed description of the invention

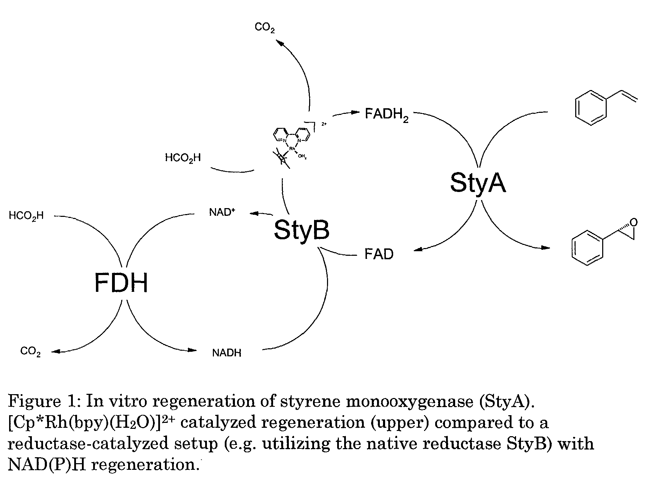

The present invention discloses a novel method to directly regenerate the oxygenase component of styrene monooxygenase for biocatalytic epoxidation of aryl- and alkyl-substiuted C-C double bonds in high enantiomeric purities (Figure 1).

Figure 1 shows the new regeneration concept for the monooxygenase component of the styrene monooxygenase enzyme system. In a traditional approach (Figure 1 lower), the reductase component (StyB) is needed to transfer reduction equivalents from NADH to the monooxygenase part. For regeneration of NADH most commonly formate dehydrogenase (FDH) with formate as stoichiometric source of reduction equivalents is used. However, [Cp*Rh(bpy)H]⁺ was shown to reduce FAD which can be applied to StyA thus eliminating the need for the reductase component, NAD and the NADH regeneration system (Figure 1 upper).

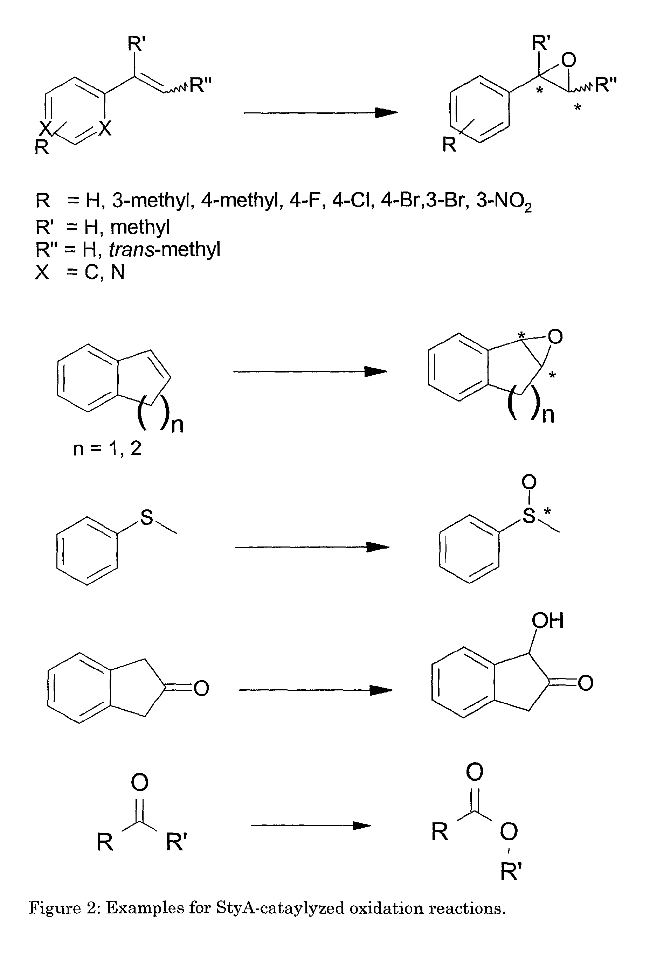

Some non-limiting examples for reactions that can be performed are given in Figure 2.

The approach outlined is not limited to the application to styrene monooxygenase but generally to any FAD-dependent monooxygenase and the reactions that it catalyzes. Some non-limiting examples for FAD-dependant oxygenases and dehydrogenases and reactions and products achievable are reactions catalyzed by: [29]

Monooxygenases comprising the E.C. number 1.14.x.y that catalyze hydroxylation reaction at aromatic rings (benzene derivatives and aromatics containing one or several heteroatoms such as O, S, N, P), epoxidation reactions of olefins, Beayer-Villiger reactions and hetero-atom oxygenations (e.g. at B, Al, Ga, N, P, As, Sb, S, Se, Te, Cl, Br, I). FAD containing oxidoreductases catalyzing the insertion of heteroatoms into organic compound such as tryptophan halogenase [30]. Dehydrogenases dependant on FAD catalyzing reduction reactions e.g. at organic acids, aldehydes, ketones, imines or C-C double bonds.

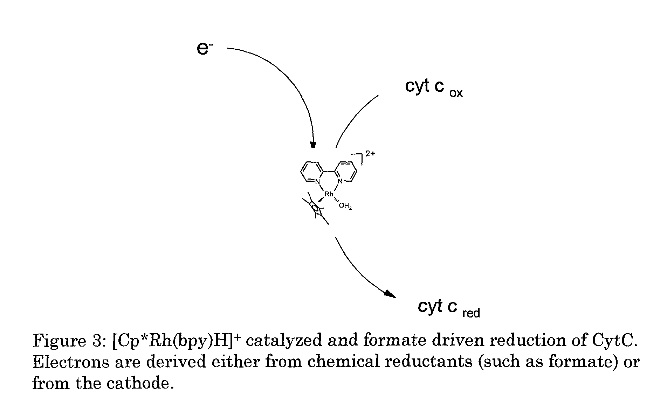

The present invention also discloses a novel method to regenerate (provide with reduction equivalents) heme- and non-heme iron enzymes such as the catalytic reduction of Cytochrome C (Figure 3).

This direct reduction of metal-containing enzymes can be applied to supply metal-containing monooxygenases with reduction equivalents necessary. Thus, the electron-transport chain (including NAD(P)H and the regeneration thereof, the reductase and the mediator protein) can be substituted by [Cp*Rh(bpy)(H₂O)]²⁺. Thus, this approach is applicable to heme- and non-heme-iron monooxygenases and dioxygenases and oxidoreductases containing other metal cations.

This invention discloses also a novel method for controllable *in situ* production of hydrogen peroxide and its coupling to P450 monooxygenases and peroxidases.

Instead of FAD, any alloxazine-based structure, reacting with molecular oxygen to hydrogen peroxide can be used (e.g. riboflavine, FMN, etc.).

By adjusting reaction parameters (especially c([Cp*Rh(bpy)(H₂O)]²⁺, c(formate), and temperature) a hydrogen peroxide formation rate can be achieved that is suitable for a given hydrogen peroxide consuming enzymatic reaction.

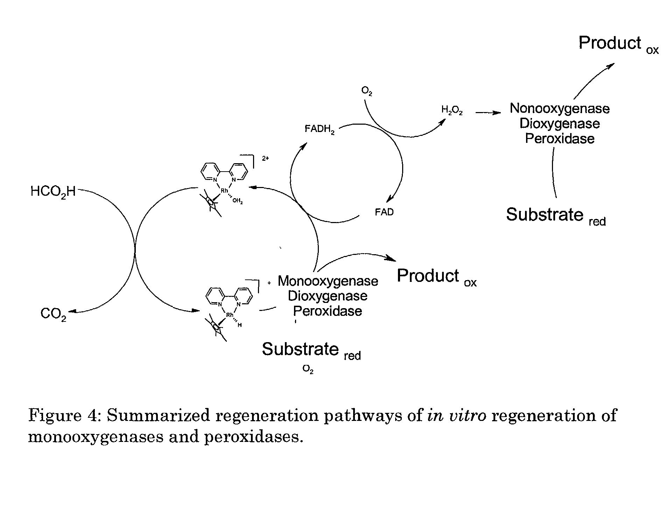

Thus, this invention discloses a general approach for the regeneration of heme-containing mono- and dioxygenases as well as peroxidase (based on heme-structures). These enzymes can be regenerated by [Cp*Rh(bpy)(H₂O)]²⁺ either by direct reduction or by utilizing the hydrogen peroxide shunt (Figure 4).

Some non-limiting examples for heme-iron containing oxygenase catalyzed reactions are:
Hydroxylation of unfunctionalized aliphatic compounds [2];
Epoxidation of alkenes [2];
Hydroxylation of aromatic compounds [2];
Dihydroxylation of aromatic compound [31].

Some non-limiting examples for non-heme-iron containing oxygenase catalyzed reactions are:
Hydroxylation of unfunctionalized aliphatic compounds [32];
Epoxidation of alkenes [32].

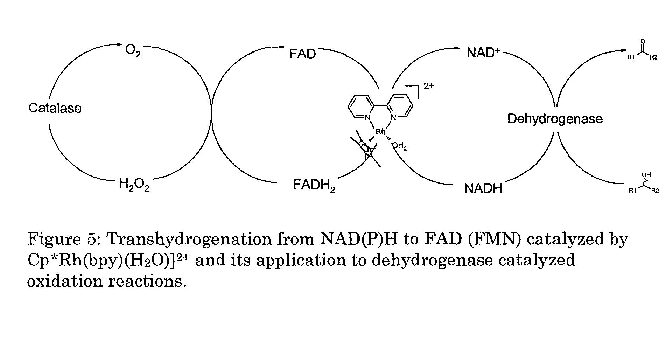

This invention also discloses a novel method for *in situ* regeneration of enzymatically active NAD(P)⁺ utilizing Cp*Rh(bpy)(H₂O)]²⁺ as oxidation catalyst and its application to dehydrogenase catalyzed oxidation reactions (Figure 5).

Thus, it can be applied e.g. to kinetic resolution of racemic alcohols leading to enantiopure alcohols or regiospecific oxidation reactions.

This invention also discloses methods to prevent the inactivation of enzymes and/or the redox-catalyst by mutual interaction.

Previously [Cp*Rh(bpy)(H₂O)]²⁺ has been reported to have no influence on the activity of several dehydrogenases (HLADH, TBADH, etc.) [13]. However, this is not generally the case for any given enzyme. For example incubation of [Cp*Rh(bpy)(H₂O)]²⁺ with 2-hydroxybiphenyl-3-monooxygenase (HbpA, E.C. 1.14.13.44) leads to complete loss of enzymatic activity. This inactivation can be prevented by utilizing nucleophilic buffers such as buffers containing ammonia or TRIS.

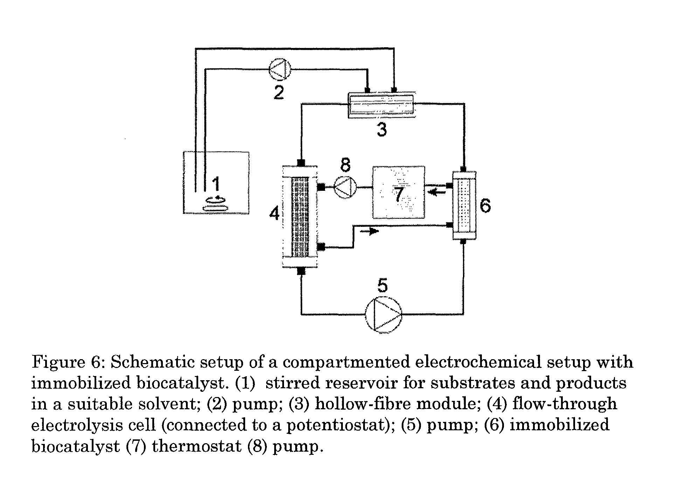

This invention also discloses a novel reactor concept to circumvent the generation of freely diffusing reactive oxygen species generated by direct reduction of molecular oxygen at the cathode (Figure 6).

One of the major problems during electrochemical reactions in O₂ containing media is the direct cathodic reduction of O₂ leading to reactive reduced oxygen species (superoxide, peroxide) that are hazardous to enzyme activity.

The biocatalyst is separated from the electrochemical cell by immobilization (e.g. on Eupergit). O₂ supply can be adjusted to a concentration in the biocatalyst compartment so that it is completely consumed by the enzymatic reaction. Thus, oxygen-free buffer is pumped through the electrolysis, and subsequently the reaction medium is lead through a hollow-fiber module to supply the medium with substrate for the enzymatic reaction and to withdraw the reaction product.

### Example 1: [Cp*Rh(bpy)H]⁺ catalyzed reduction of cytochrome C (cytC)

In a typical experiment potassium phosphate buffer (50 mM, pH 7.0, c(NaHCO₂) = 150 mM) is supplemented with cytC- (from horse heart, Fluka, Buchs Switzerland) and [Cp*Rh(bpy)(H₂O)]²⁺- stocksolutions to 1 ml final volume in a quartz kuvette and placed into a thermostatted (T = 30°C ) UV-spectrophotometer (UNICAM). After addition of the components, the absorption at λ = 550 nm is followed. Concentrations of reduced CytC were determined after background correction according to the law of Lamber-Beer utilizing a molar extinction coefficient for reduced CytC of 21000 cm² * mol⁻¹.

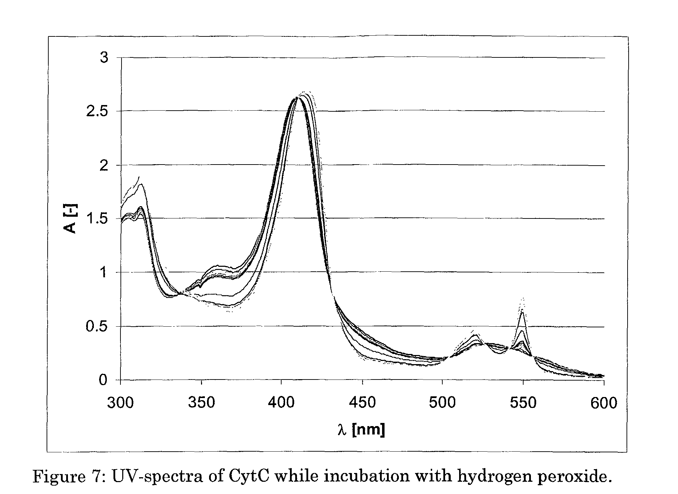

Figure 7 displays the UV spectra recorded at intervals of 1 minute during one typical experiment.

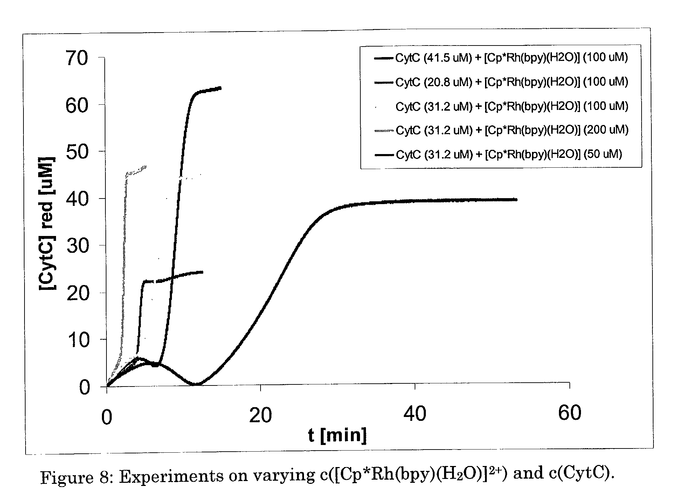

Maximum absorptions at 550 nm (originating from the absorption of reduced cytC) Figure 8 correlate independently from c([Cp*Rh(bpy)(H₂O)]²⁺ linearly to the concentration of cytC. Duration of the lack phase and rate of cytC reduction however correlate to c([Cp*Rh(bpy)(H₂O)]²⁺).
**Result:** Heme-containing enzymes (represented by CytC) can be reduced by reduced [Cp*Rh(bpy)H]⁺ *(in situ* regenerated by formate). Quantitative reduction of the protein can be achieved.

Analogous experiments were carried out in the absence of the catalyst ([Cp*Rh(bpy)(H₂O)]²⁺), the source of reduction equivalents (NaHCO₂) and cytC. None of these controls lead to significant increase in the absorption at 550 nm.
**Result:** These controls prove [Cp*Rh(bpy)(H₂O)]²⁺ catalyzed reduction of CytC.

In another typical experiment, the reaction temperature was varied between 20 and 35 °C.

Typical reaction parameters are given in Table 1.

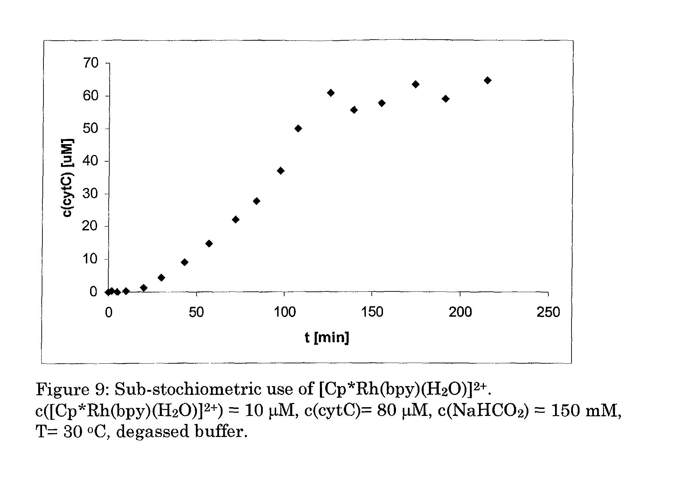

To exclude stoichiometric interaction of [Cp*Rh(bpy)(H₂O)]²⁺ with cytC, in another experiment low c([Cp*Rh(bpy)(H₂O)]²⁺) were applied. In 50 ml degassed potassium phosphate buffer (50 mM, pH 7.0, c(NaHCO₂) = 150 mM) 10 µM ([Cp*Rh(bpy)(H₂O)]²⁺) were incubated with 80 µM cytC (Figure 9).
**Result:** Multiple turnovers of [Cp*Rh(bpy)(H₂O)]²⁺ as reduction catalyst can be obtained. Turnover frequencies for [Cp*Rh(bpy)(H₂O)]²⁺ are in the range of 4―32 turnovers per hour and depend on the reaction temperature. The total turnover number of 6 for [Cp*Rh(bpy)(H₂O)]²⁺ the example given in Figure 9 is not optimized yet.

### Example 2: [Cp*Rh(bpy)H]⁺ catalyzed regeneration of the monooxygenase component of styrene monooxygenase (StyA)

In a typical experimental setup 1 ml total volume was placed in an Eppendorf cup in a Thermomixer. The temperature was set to 37°C and the reaction mixture was shaken thoroughly to ensure O₂ intake. General compositions are given in Table 2:
Product formation was followed by reversed phase HPLC. The HPLC system (Merck) consisted of a D-7000 controller, L-7200 autosampler, L-7400 UV detector, L-7100 liquid chromatography pump and was fitted with a CC250/4 Nucleosil 100-5 C18 HD column. Samples were eluted under isocratic conditions using 60% acetonitrile and 40% water at a flow rate of 1 ml × min⁻¹. The elution pattern was monitored at 210 nm.
Hydrogen peroxide was determined enzymatically based on the method by Saito et al. [33]. Samples were mixed with a solution of 0.6 mM 4-amino antipyrine, 9 mM phenol, and 6 U × ml⁻¹ peroxidase from *Coprinus cinereus.* After one minute incubation at room temperature the absorption at 550 nm was measured.

### Experiment 1: Controls

Experiments leaving out either [Cp*Rh(bpy)(H₂O)]²⁺ or StyA yielded no detectable formation of styrene oxide. Also the incubation of hydrogen peroxide in the presence of StyA and FAD did not lead to the formation of styrene oxide.
**Result:** Reaction mechanisms other than outlined in Figure 1 (upper) can be excluded.

### Experiment 2: Styrene oxide formation over time

0.2 mM [Cp*Rh(bpy)(H₂O)]²⁺, 0.05 mM FAD, 0.005 mM FMN, 2 mM styrene, 280 U catalase, and 1.16 µM StyA in 50 mM potassium phosphate buffer with 150 mM sodium formate were incubated at 37°C and agitation at 1500 rpm. At intervals, reactions were stopped by addition of one equivalent volume of cold acetonitrile. After centrifugation (15 min at 4°C and 23000 × g the upper phase was analyzed by reverse phase HPLC. Concentrations were corrected according to the phase ratio after centrifugation. Results from this experiment are given in Figure 10 and Table 3.

In a second experiment FMN was left out from the reaction mixture without detectable loss in styrene oxide productivity.
**Result:** The highly simplified regeneration approach for StyA (as member of FAD-dependant monooxygenases) is functional. Multiple turnovers for both the enzyme and the regeneration catalyst can be achieved in the simplified *in vitro* regeneration approach (Figure 1).

### Experiment 3: Styrene as organic phase

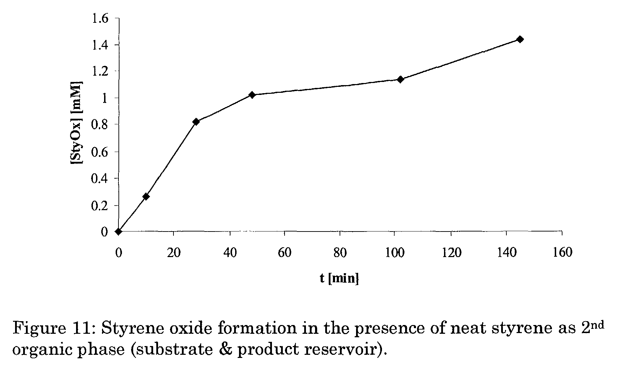

A total volume of 500 µl 0.2 mM [Cp*Rh(bpy)(H₂O)]²⁺, 0.033 mM FAD, FMN, 280 U catalase, and 1.16 µM StyA in 50 mM potassium phosphate buffer with 150 mM sodium formate was incubated with 500 µl styrene (2 mM dodecane as internal standard) at 37°C and agitation at 1500 rpm. At intervals, samples from the styrene phase were withdrawn and analyzed by GC. Results from this experiment are given in Figure 11.
**Result:** Biphasic reaction media containing the neat substrate as substrate-& product reservoir can be applied. Initial productivities are in the range of one-phase reactions.

### Experiment 4: The role of non-StyA supported reduction of FAD (leading to hydrogen peroxide)

0.2 mM [Cp*Rh(bpy)(H₂O)]²⁺, 0.05 mM FAD, and 1.16 µM StyA in 50 mM potassium phosphate buffer with 150 mM sodium formate were incubated at 37°C and agitation at 1500 rpm. At intervals samples were taken and analyzed for their hydrogen peroxide concentration. From the results a catalytic performance of 48 turnovers per hour for [Cp*Rh(bpy)(H₂O)]²⁺ was calculated.

In a second experiments a reaction mixture of this composition was supplemented with 2 mM styrene. After 15 min the reaction was stopped and analyzed for hydrogen peroxide and styrene oxide. 0.28 mM styrene oxide and 2.07 were found at this time corresponding to an overall catalytic activity of [Cp*Rh(bpy)(H₂O)]²⁺ of 47 turnovers per hour.

### Experiment 5: Variation of catalyst concentrations

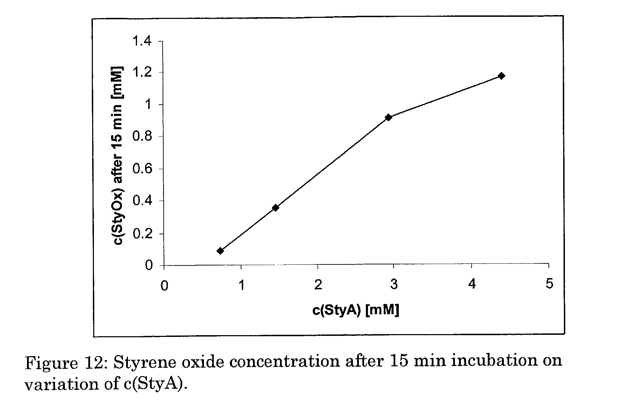

0.4 mM [Cp*Rh(bpy)(H₂O)]²⁺, 0.05 mM FAD, 4 mM styrene, 280 U catalase, and 0.1 - 4.4 µM StyA in 50 mM potassium phosphate buffer with 150 mM sodium formate were incubated 15 minutes at 37°C and agitation at 1500 rpm (Figure 12).

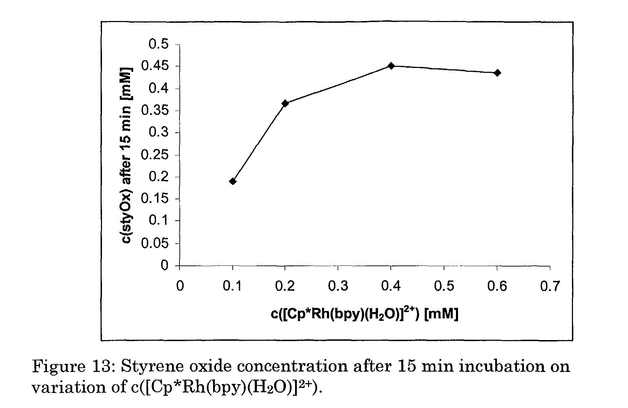

In a second experiment, 0.1― 0.6 mM [Cp*Rh(bpy)(H₂O)]²⁺, 0.05 mM FAD, 4 mM styrene, 280 U catalase, and 1.47 µM StyA in 50 mM potassium phosphate buffer with 150 mM sodium formate were incubated 15 minutes at 37°C and agitation at 1500 rpm (Figure 13).

### Experiment 6: The role of FAD concentration on styrene oxide formation

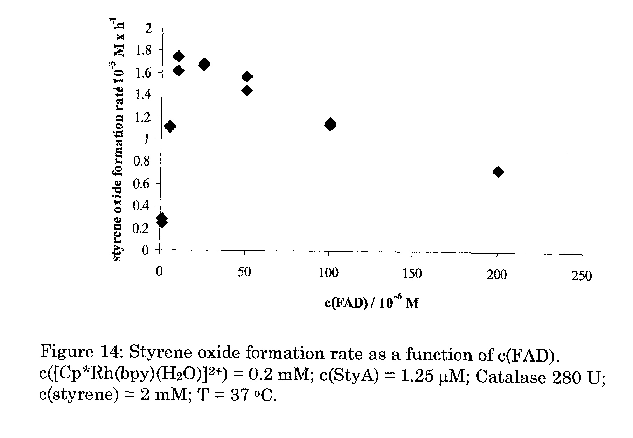

0.2 mM [Cp*Rh(bpy)(H₂O)]²⁺, 280 U catalase, 1.25 µM StyA, 2 mM styrene, and 1-200 µM FAD in 50 mM potassium phosphate buffer with 150 mM sodium formate were incubated for 15 minutes at 37°C and agitation at 1500 rpm. Afterwards the reaction mixtures were analyzed by reverse phase HPLC for their styrene oxide concentration (Figure 14).
**Result from experiments 4-6:** Non-StyA supported reduction of FAD is the main competing reaction. However, this undesired reaction can be minimized by engineering of the reaction conditions. Preliminary maximum space-time yield are as high as 4.8 mM/(l*h).

### Experiment 7: Substrate range and enantioselectivity

0.2 mM [Cp*Rh(bpy)(H₂O)]²⁺, 0.02 mM FAD, 2 mM substrate, 200 U catalase, and 1.47 µM StyA in 50 mM potassium phosphate buffer with 150 mM sodium formate were incubated at 37°C and agitation at 1500 rpm.

Styrene oxide was analyzed using a CC200/4 Nucleodex α-PM (Machery-Nagel). As eluent 60% methanol and 40 % 6.2 mM TEAA in water was used at a flow rate of 0.7 ml × min⁻¹. 1,2-Dihydronaphthalene oxide, trans-β-Methylstyrene oxide, indene oxide, and methyl phenyl sulfoxide were extracted from the reaction buffer with one aliquot hexane und analyzed unsing normal phase HPLC with a CHIRACEL OB-H column and hexane:isopropanol 95:5 (9:1 for methyl phenyl sulfoxide) at a flow rate of 0.5 ml*min⁻¹ as mobile phase. As reference either authentical samples of racemic epoxides were used. (Table 4).
**Result:** The simplified regeneration approach does not limit the substrate range of StyA catalyzed epoxidation reactions nor lowers it the enantiospecificity of the epoxidation reaction. Furthermore, sulfoxidations catalyzed by StyA have not been reported yet.

### Experiment 8: Immobilization of StyA

1 ml StyA solution (2.2 mg * ml⁻¹) are incubated with 200 mg Eupergit C™ at 4°C for 3 days. Afterwards the immobilisate is washed to remove unbound proteins and stored in 50 mM phosphate buffer (pH 7) at 4°C.

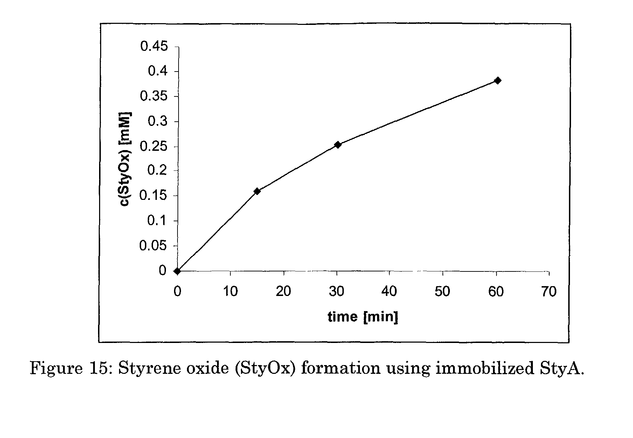

In a typical experiment 100 mg of the immobilisate are incubated in potassium phosphate buffer (50 mM, pH 7.0) with 150 mM sodium formate, 50 µM FAD, 2 mM styrene, and 0.2 mM [Cp*Rh(bpy)(H₂O)]²⁺ at 37°C and 1500 rpm. At intervals, samples are taken from the supernatant and analyzed by reverse phase HPLC (Figure 15).
**Result:** StyA covalently bound to a solid matrix is catalytically active. Enzyme activities on the solid matrix are considerably lower than freely diffusing enzyme (about 10 ― 20%) but can be optimized by advanced immobilization procedures and materials.

### Example 3: [Cp*Rh(bpy)H]⁺ catalyzed formation of hydrogen peroxide coupled to heme-containing enzymes

Hydrogen peroxide produced was determined enzymatically with a modified assay by method by Saito et al. [33]. Samples were mixed with a solution of 0.6 mM 4-amino antipyrine, 9 mM phenol, and 6 U × ml⁻¹ peroxidase from *Coprinus cinereus.* After one minute incubation at room temperature the absorption at 550 nm was measured.

By using hydrogen peroxide samples of known concentration, a calibration curve was generated.

FADH₂ concentrations were determined on the depleting absorption of FAD at λ = 450 nm using a molar extinction coefficient of 12000 M⁻¹ cm⁻².

Oxygen measurements were performed polarographically with an oxygen electrode, which was mounted to a gas-tight thermostatted 2 ml reaction vessel. Prior to experiments, calibration of the electrode was performed in oxygen-saturated buffer and O₂-free buffer.

### Experiment 1: Depletion of molecular oxygen and accumulation of reduced FADH₂

0.2 mM [Cp*Rh(bpy)(H₂O)]²⁺, 0.225 mM FAD dissolved in O₂ saturated potassium phosphate buffer were thermostated at 37°C in an air-tight reaction vessel (O₂-measuremnts: 2ml, UV-measurements: 1ml quartz kuvette). Reactions were started on addition of sodium formate stock solution (final concentration: 150 mM).

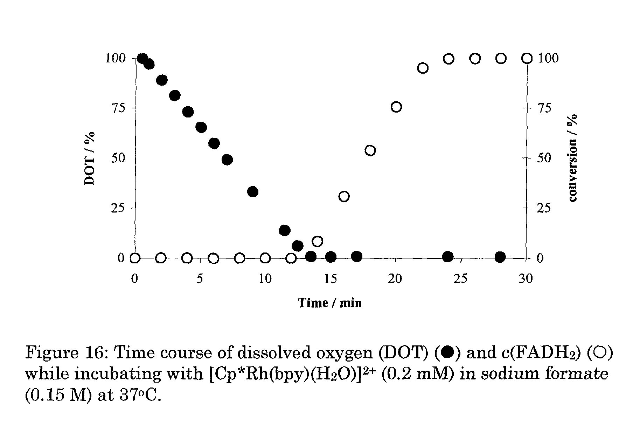

Figure 16 shows the time course of O₂-depletion and subsequent accumulation of reduced FADH2 under reaction conditions where diffusion of O₂ into the reaction medium is prevented.
**Result:** FADH₂ can be *in situ* produced by [Cp*Rh(bpy)H]⁺. It reacts very fast (diffusion limitation) with molecular oxygen.

### Experiment 2: Continuous generation of hydrogen peroxide

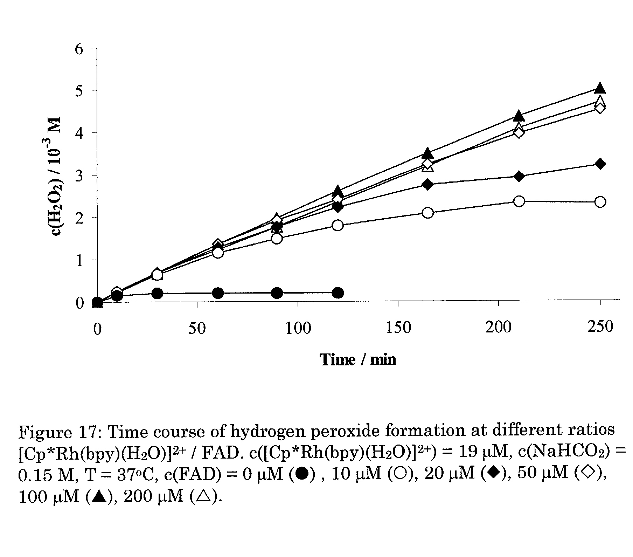

1 ml of 19 µM Cp*Rh(bpy)(H₂O)]²⁺ and 0―200 µM FAD in 50 mM potassium phosphate buffer (pH 7.0, c(NaHCO₂) = 150 mM) are placed in a 1.5 ml Eppendorf vial and incubated at 37°C and agitation at 1500 rpm. At intervals 100 ml samples are withdrawn and analyzed for their H₂O₂ content (Figure 17).

Initial H₂O₂-formation rates correspond to catalytic activities for [Cp*Rh(bpy)(H₂O)]²⁺ of 69.9 ± 2.5 catalytic cycles per hour (37°C, c(NaHCO₂) = 0.15 M). These values were obtained from Experiment 1 (oxygen consumption and FADH₂ accumulation) as well. The reaction rate depends linearly on c([Cp*Rh(bpy)(H₂O)]²⁺).

In analog experiments FAD was substituted by FMN leading to identical results.
**Result:** The formation rate of hydrogen peroxide can be adjusted by varying the [Cp*Rh(bpy)(H₂O)]²⁺ concentration and are (at first) independent from the concentration of FAD (FMN). However, over longer time scales ratios of FAD/[Cp*Rh(bpy)(H₂O)]²⁺ higher than 1 are required to maintain [Cp*Rh(bpy)(H₂O)]²⁺ activity.

### Experiment 3: CytC as P450-like protein; its inactivation by hydrogen peroxide

As described above, CytC exhibits monooxygenase activity in the presence of hydrogen peroxide (hydrogen peroxide shunt). On the other hand CytC is inactivated by hydrogen peroxide, which can be followed by the disappearing Soret-peak (heme absorbance at λ = 408 nm) [24].

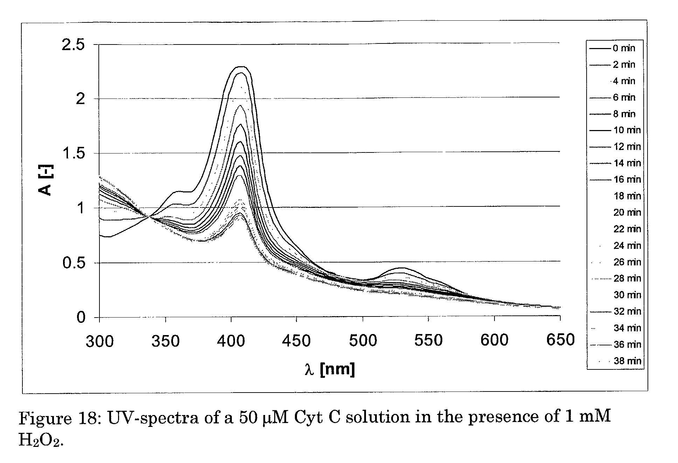

In a typical experiment 50 mM CytC is incubated at 30°C in the presence of 1 mM hydrogen peroxide. At intervals of 2 min the UV-spectrum (between 300 and 650 nm) is recorded (Figure 18).

In identical experiments, the absorbance at 408 nm was followed over time. Table 5 gives the absolute rates of the decreasing absorption at 408 nm in the linear phase at varying CytC:H₂O₂ ratios.

After preincubation of CytC (15 µM) in potassium phosphate buffer in the presence of 1 mM hydrogen peroxide for 10 minutes lead to almost complete loss of enzymatic activity (measured on the sulfoxidation of thioanisol where only traces of methyl phenyl sulfoxide were detectable).
**Result:** CytC (representing the class of P450-like monooxygenases) is rapidly inactivated by hydrogen peroxide. This inactivation is a bimolecular process; the inactivation rate linearly depends on c(H₂O₂) as well as on c(CytC). Therefore, stochiometric addition of hydrogen peroxide is not applicable to preparative scale applications.

### Experiment 4: Coupling of [Cp*Rh(bpy)(H₂O)]²⁺ catalyzed in situ production of hydrogen peroxide to CytC catalyzed oxidation of thioanisol.

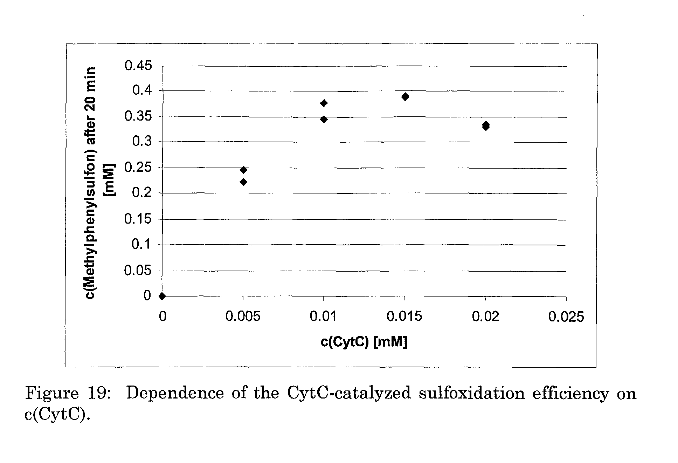

In a typical experiment 1 ml of 200 µM [Cp*Rh(bpy)(H₂O)]²⁺, 50 µM FAD, 2 mM thioanisol, and 5-20 µM CytC in 50 mM potassium phosphate buffer (pH 7.0, c(NaHCO₂) = 150 mM) are placed in a 1.5 ml Eppendorf vial and incubated at 37°C and agitation at 1500 rpm. After 20 minutes the reactions are stopped by addition of 10 µl perchloric acid (10%), centrifuged and the supernatant analyzed by reversed phase HPLC (Figure 19).

In an otherwise identical experiment c(CytC was set to 20 µM whereas the concentration of [Cp*Rh(bpy)(H₂O)]²⁺ was varied between 100 µM and 400 µM. A linear dependence of product concentration on the concentration of [Cp*Rh(bpy)(H₂O)]²⁺ was found.

1 ml of 200 µM [Cp*Rh(bpy)(H₂O)]²⁺, 50 µM FAD, 2 mM thioanisol, and 83 µM CytC in 50 mM potassium phosphate buffer (pH 7.0, c(NaHCO₂) = 150 mM) are placed in a 1.5 ml Eppendorf vial and incubated at 37°C and agitation at 1500 rpm. After 20 minutes the reactions are stopped by addition of 10 µl perchloric acid (10%), centrifuged and the supernatant analyzed by reversed phase HPLC. Experiments leaving out either CytC or [Cp*Rh(bpy)(H₂O)]²⁺ under otherwise identical conditions yielded no detectable formation of methyl-phenyl sulfoxide from thioanisol.

In the presence of 560 U catalase yielded in the formation of traces of methyl-phenyl sulfoxide (> 0.01 mM after 20 minutes).

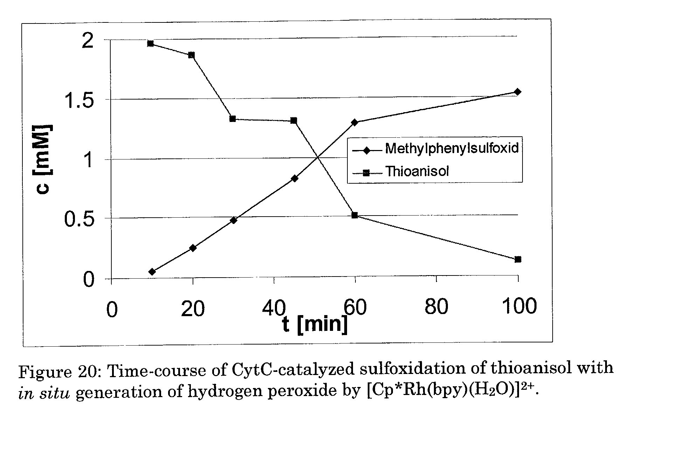

In another experiment 1 ml of 200 µM [Cp*Rh(bpy)(H₂O)]²⁺, 50 µM FAD, 2 mM thioanisol, and 20 µM CytC in 50 mM potassium phosphate buffer (pH 7.0, c(NaHCO₂) = 150 mM) are placed in a 1.5 ml Eppendorf vial and incubated at 37°C and agitation at 1500 rpm. At intervals, samples are withdrawn and analyzed by reversed phase HPLC (Figure 20).
**Result:** The [Cp*Rh(bpy)(H₂O)]²⁺ catalyzed formation of hydrogen peroxide is applicable to supply a P450-like monooxygenase with an appropriate amount of hydrogen peroxide for optimal stability and productivity. Thus, long-term oxidation reactions become available.

### Example 4: Interaction of [Cp*Rh(bpy)(H₂O)]²⁺ with proteins

### Analytical method:

HbpA-activities were determined by UV-spectroscopy according to literature methods [34] by supplementing the experiment buffer with 0.1 mM NADH and observation of NADH-depletion at 340 nm for 1 minute, afterwards 2 mM of 2-hydroxybiphenyl were added and NADH depletion was measured for 1 minute. HbpA activity was determined as difference of both rates.

[Cp*Rh(bpy)(H₂O)]²⁺ activity was determined by UV-spectroscopy by supplementing the experiment buffer with 0.1 mM NAD⁺ and 150 mM final concentration of sodium formate and following the NADH formation at 340 nm.

### Experiment 1: Mutual inactivation of 2-hydroxybiphenyl-3-monooxygenase (HbpA. E.C. 1.14.13.44) and [Cp*Rh(bpy)(H₂O)]²⁺

In a first experiment 0.11 U * ml⁻¹ HbpA of a partially enriched crude extract from recombinant E. *coli* overexpressing HbpA was incubated at 30°C with 0, 0.02, 0.04, and 0.1 mM [Cp*Rh(bpy)(H₂O)]²⁺. In a second experiment c[Cp*Rh(bpy)(H₂O)]²⁺) was maintained at 0.02 mM whereas the HbpA content varied form 0.22 U * ml⁻¹ to 1.52 U * ml⁻¹. At intervals, samples were analyzed with respect to residual HbpA activity Table 6 displays half-live times and residual HbpA activities after 60 minutes of incubation.
**Result:** [Cp*Rh(bpy)(H₂O)]²⁺ can interact with proteins leading to loss of catalytic activity of both enzyme and [Cp*Rh(bpy)(H₂O)]²⁺. The inactivation process is stochiometric.

### Experiment 2: Prevention of HbpA-inactivation by [Cp*Rh(bpy)(H₂O)]²⁺ by blocking the Rh-coordination sphere with NH₃

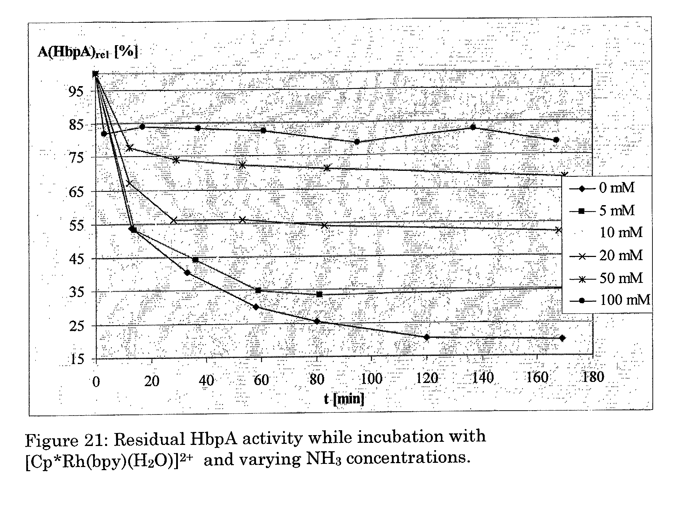

0.28 U * ml⁻¹ HbpA were incubated at varying ammonia concentrations (0 - 100 mM) with 0.04 mM [Cp*Rh(bpy)(H₂O)]²⁺. At intervals, samples were examined towards their residual enzyme activity (Figure 21).
**Result:** Since the nature of mutual inactivation of [Cp*Rh(bpy)(H₂O)]²⁺ and enzyme is coordination of nucleophilic groups at the enzymes surface, it can be prevented by blocking the free coordination space at the rhodium ion with nucleophilic ligands such as NH₃.

Experiment 3: [Cp*Rh(bpy)(H₂O)]²⁺ activity in the presence of 100 mM NH₄± under chemical and electrochemical reduction

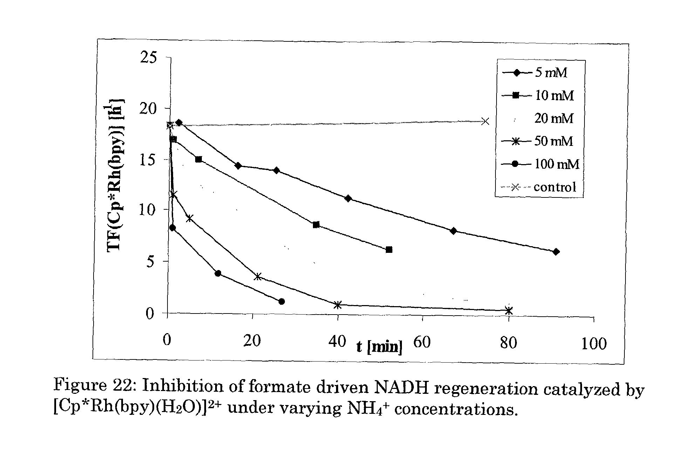

0.04 mM [Cp*Rh(bpy)(H₂O)]²⁺ in 50 mM potassium phosphate buffer (50 mM, pH 7.5) are incubated at 30°C with NH₄⁻ concentrations of 0, 5, 10, 20, 50, 100 mM. At intervals, samples are analyzed by UV-spectroscopy towards their activity under formate driven NADH regeneration. Assay conditions were: 500 µl sample are given to 500 µl (2 mM NAD⁺, 150 mM NaHCO₂, T = 30°C): the absorption at 340 nm is followed for 1 minute (Figure 22).

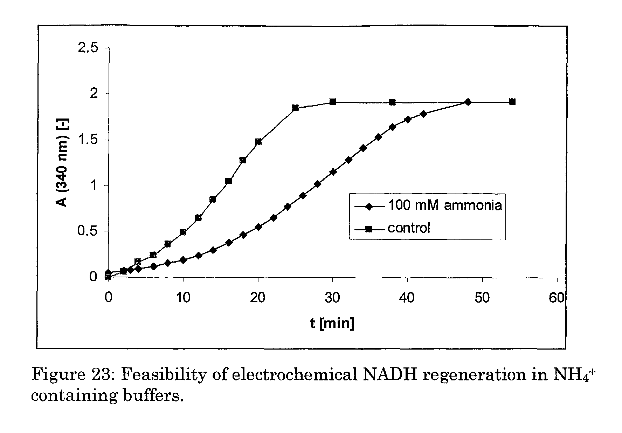

In another experiment, 0.02 mM [Cp*Rh(bpy)(H₂O)]²⁺ at 30°C in 50 mM potassium phosphate buffer (c(NAD⁺) = 1 mM) either in the presence or absence of 100 mM NH₄⁺ is reduced cathodical at a carbon-felt electrode (E = - 750 mV vs. Ag/AgCl sat.) (Figure 23).
**Result:** Even though NH₃ containing buffers inhibit the formate-driven regeneration of [Cp*Rh(bpy)H]⁺ (and thus the NADH regeneration), electrochemical regeneration of NADH is possible.

### Example 5: Regeneration of oxidized nicotinamide coenzymes utilizing [Cp*Rh(bpy)(H₂O)]²⁺ as transhydrogenation catalyst between NAD(P)H and FAD

In a typical experiment, a 0.1 mM NADH solution in 50 mM potassium phosphate buffer (50 mM , pH 7.0) is placed in a kuvette in a UV-spectrophotometer. The reaction mixture is thermostatted at 30°C. After supplementing with [Cp*Rh(bpy)(H₂O)]²⁺ (0-0.02 mM) and FAD (0-0.04 mM) the absorption at 340 nm is followed.

The initial rate is linearly dependent on c([Cp*Rh(bpy)(H₂O)]²⁺). FAD can be substituted by FMN.
**Result:** [Cp*Rh(bpy)(H₂O)]²⁺ serves as catalyst in the transhydrogenation reaction between reduced nicotinamide coenzymes and alloxazine-based structures (such as FAD or FMN) in the presence of molecular oxygen the oxidized alloxazine is regenerated very fast (see also Figure 16). This can serve as regeneration concept for oxidized nicotineamide coenzymes (NAD(P)⁺) from their reduced forms.

### Application to a NADH-consuming enzymatic reaction

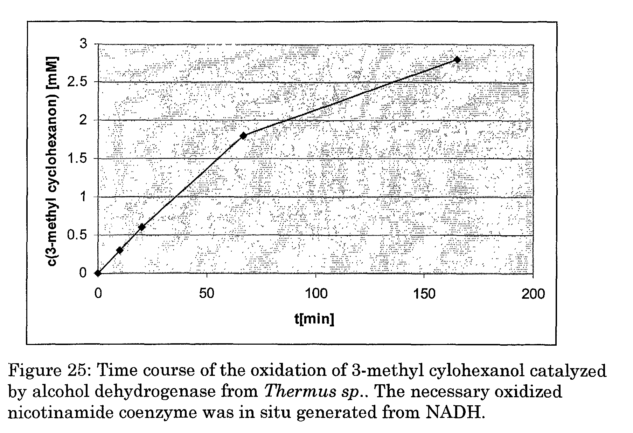

In a typical experiment, 20 mM of 3-methyl cyclohenanol were incubated with 5 mM NADH, 50 µM [Cp*Rh(bpy)(H₂O)]²⁺, 200 µM FAD, 10000 U catalase and 38 nags of alcohol dehydrogenase from *Thermus sp.* In 50 mM potassium phosphate buffer at 60 °C. At intervals, samples were withdrawn and analyzed by gas chromatography (Figure 25).

In a control experiment under identical conditions and in the absence of TADH, only traces of 3-methyl cyclohexanone were detectable.
**Result:** The proposed regeneration approach for oxidized nicotinamide coenzymes is applicable to enzymatic reaction (here represented by the dehydrogenase from *Thermus sp.).*

### Example 6: Direct reduction of alkane hydoxylase from P. Oleovorans (AlkB)

In a typical experiment, 1 ml total volume of 200 µM [Cp*Rh(bpy)(H₂O)]²⁺ are incubated with 2 mM 3-ethyl toluene and 100 µl of a cell crude extract of recombinant *E. coli* overexpressing AlkB in a final volume of 1 ml 100 mM potassium phosphate buffer (pH 7.4) supplemented with 150 mM sodium formate and 1 mg/ml bovine serum albumin at 30 °C and vigorous shaking. After 20 minutes the reaction is stopped by addition of 10 ml perchloric acid (10%). After centrifugation the supernatant is extracted with one aliquot of hexane. The organic phase is analyzed by normal phase HPLC (Nucleosil 250/3 100-5 C, mobile phase: hexane/isopropanol 95:5, flow rate = 0.5 ml min⁻¹).

From comparison with authentical samples, about 0.015 mM (average of 4 experiments) of 3-methylphenethylalcohol were produced.

**Table 1:**

| CytC reduction rates at varying temperatures. (c(CytC) =31.2 µM, c(NaHCO₂) = 150 mM) | | | |
|---|---|---|---|
| T [°C] | c[Cp*Rh(bpy)(H₂O)]²⁺) [µM] | dc(CytC)^{red} / dt [mM*h⁻¹] | TF ([Cp*Rh(bpy)(H₂O)]²⁺) [h⁻¹] |
| 20 | 100 | 0.4 | 4 |
| 30 | 100 | 2.1 | 21 |
| 35 | 50 | 1.6 | 32 |

**Table 2:**

| Typical reaction conditions. | |
|---|---|
| Compound | Conentration |
| [Cp*Rh(bpy)(H₂O)]²⁺ | 0 ― 0.4 mM |
| FAD | 0 ― 0.2 mM |
| NaHCO₂ | 0 ― 150mM |
| [StyA] | 0―4 µM |
| [Substrate] | 0 - saturation |
| Catalase | 0―1000 U*ml⁻¹ |
| Reaction time | 5 ― 180 min |

**Table 3:**

| Characteristics for the chemo-enzymatic epoxidation reaction represented in Figure 10 | |
|---|---|
| Parameter | Value |
| Conversion / % | 100 |
| Yield / % | 53 ^{a} |
| Space-time yield [10⁻³ mol × 1⁻¹ × h⁻¹] | 2.26 |
| StyA performance (TF / TTN)^{b} | 0.54 s⁻¹ / 914 |
| [Cp*Rh(bpy)(H₂O)]²⁺ performance (TF / TTN)^{b} | 11.3 h⁻¹ / 5.3 |
| relative activity / % | 16.1^{c} |

| | |
|---|---|
| a due to high volatility of styrene. | |
| b TTN = total product concentration × catalyst concentration⁻¹; TF = TTN × time⁻¹. | |
| c calculated on FADH₂ generation rate determined in the absence of StyA. | |

**Table 4:**

| Substrates. (Reaction conditions: T = 37 °C, c([Cp*Rh(bpy)(H₂O)]²⁺) = 0.2 mM, c(StyA) = 1.47 µM, c(NaHCO₂) = 150 mM, c(FAD) = 20 µM, c(Catalase) = 200 U*ml⁻¹. | | | |
|---|---|---|---|
| Substrate (2 mM) | Reaction time [min] | Epoxides concentration [mM] | ee-value of the epoxides [%] |
| Styrene (4 mM) | 15 | 0.33 | < 98 (S) ^{[a]} |
| 1,2-Dihydronaphthalene | 15 | 0.23 | 98.9 |
| Trans-β-Methylstyrene | 15 | 0.28 | 97.6 |
| Indene | 15 | 0.42 | 97.9 |
| 3-Chloro-styrene | 15 | 0.37 | n.d.^{[b]} |
| 4-Methyl-styrene | 15 | 0.29 | n.d ^{[b]} |
| 4-Fluoro styrene | 15 | Positive ^{[c]} | n.d. ^{[b]} |
| 4-Chloro styrene | 15 | Positive ^{[c]} | n.d. ^{[b]} |
| 4-Bromo styrene | 15 | Positive ^{[c]} | n.d. ^{[b]} |
| 4-Methoxy styrene | 15 | Positive ^{[c]} | n.d. ^{[b]} |
| Thioanisole | 15 | 0.33 (Methyl phenyl sulfoxide) | 23.3 |
| Phenyl ethyl sulfide | 15 | Positive ^{[c]} | n.d. ^{[b]} |

| | | | |
|---|---|---|---|
| a: confirmed by authentical samples | | | |
| b: not determined yet | | | |
| c: no standard substances available yet | | | |

**Table 5:**

| CytC inactivation as a function of [CytC] and [H₂O₂]. | | |
|---|---|---|
| [CytC] [µM] | [H₂O₂] [mM] | dE(408 nm)/dt [min-1] |
| 10 | 0 | 0 |
| 10 | 5 | -0.0234 |
| 10 | 7.5 | -0.0330 |
| 10 | 10 | -0.0438 |
| 10 | 15 | -0.0637 |
| 10 | 20 | -0.0821 |
| 5 | 5 | -0.0116 |
| 5 | 10 | -0.0231 |
| 5 | 20 | -0.0399 |
| 7.5 | 10 | -0.0281 |

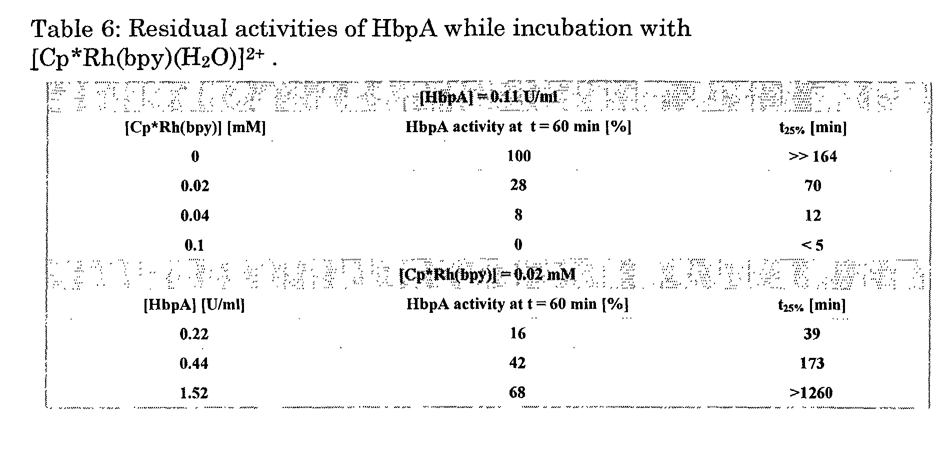

### References

1. Adam, W., et al. in Advances in Biochemical Engineering /Biotechnology, K. Faber, Editor. 1999, Springer: Berlin, Heidelberg. p. 74-104.
2. Drauz, K.-H. and H. Waldmann, Enzyme Catalysis in Organic Synthesis. 2 ed. 2002, Weinheim: Wiley-VCH.
3. Steckhan, E., et al., 2002. Patent Nr. WO0236794
4. Schmid, A., et al. Journal of Molecular Catalysis B: Enzymatic, 2001. 11: p. 455-462.
5. Rissom, S., et al. Tetrahedron: Asymmetry, 1997. 8(15): p. 2523-2526.
6. Gagnon, R.G., Gideon; Roberts, Stanley M.; Villa, Raffaella; Willetts, Andrew J., Journal of the Chemical Society Perkins Transactions, 1995: p. 1505-1511.
7. Gagnon, R., et al., Journal of the Chemical Society Perkins Transactions, 1994: p. 2537-2543.
8. Willetts, A.J., et al., Journal of the Chemical Society Perkins Transactions, 1991: p. 1608-1610.
9. Bastos, F., et al., Biotechnology Techniques, 1999. 13: p. 661-664.
10. Kim, Y., et al., Journal of Biotechnology, 1998. 59: p. 213-220.
11. Leonida, M.D. Current Medical Chemistry, 2001. 8: p. 345-369.
12. Hollmann, F., B. Witholt, and A. Schmid, Journal of Molecular Catalysis B: Enzymatic, 2002: p. accepted.
13. Westerhausen, D., et al., Angewandte Chemie International Edition in English, 1992. 31(11): p. 1529-1531.
14. Ruppert, R., S. Herrmann, and E. Steckhan Tetrahedron Letters, 1987. 28(52): p. 6583-6586.
15. Hollmann, F., A. Schmid, and E. Steckhan Angewandte Chemie International Edition in English, 2001. 40(1): p. 169-171.
16. Schmid, A., et al. 2001. Patent Nr. WO0188172
17. Reipa, V., M.P. Mayhew, and V.L. Vilker, Proceedings of the National Academy of Science of the United States of America, 1997. 94: p. 13554-13558.
18. Mayhew, M.P., et al., Biotechnology Progress, 2000. 16: p. 610-616.
19. Hauer, B., R.D. Schmid, and U. Schwaneberg, 2001. Patent Nr. WO 0107573
20. Faulkner, K.M., et al. Proceedings of the National Academy of Science of the United States of America, 1995. 92: p. 7705-7709.
21. Kazlauskaite, J., et al., Chemical Communication, 1996: p. 2189-2190.
22. Gosling, P.A. and R.J.M. Nolte, Journal of Molecular Catalysis A: Chemical, 1996. 113(113): p. 257-267.
23. Joo, H., Z. Lin, and F.H. Arnold, Nature, 1999. 399: p. 670-673.
24. Vazquez-Duhalt, R., Journal of Molecular Catalysis B: Enzymatic, 1999. 7(1-4): p. 241-249.
25. Katakis, I.D., E., Mikrochim. Acta, 1997. 126: p. 11-32.
26. Hilt, G.J., T.; Heineman, W.R.; Steckhan, E. Chem. Eur. J., 1997. 3: p. 79-88.
27. Jones, J.B. and K.E. Taylor, Canadian Journal of Chemistry, 1976. 54(19): p. 2969-2973.
28. Kölle, U.R., A.D., Mendeleev Commun., 1995: p. 187-189.
29. Palfey, A. and V. Massey in Comprehensive Biological Catalysis, M. Sinnott, Editor. 1998, Academic Press: San Diego, London. p. 83-154.
30. Hoelzer, M., et al., Advances Synthesis and Catalysis, 2001. 6-7: p. 591-595.
31. Schonfield, J.A. 1989. Patent Nr. US 4,8889,804
32. Wubbolts, M.G., et al., Chimia, 1996. 50: p. 439-437.
33. Saito, Y., et al., Talanta, 1987. 34(7): p. 667-669.
34. Suske, W.A.H., M.; Schmid, A.; Fleischmann, T.; Wubbolts, M.G.; Kohler, H.-P. E.; Journal of Biological Chemistry, 1997. 272(39): p. 24257-24265.

## Claims

1. A process for the specific oxygenation of an organic compound by contacting this compound with molecular oxygen in the presence of a cell-free microbial monooxygenase.

2. The process of claim 1, wherein the oxygenation reaction comprises one of the reactions of Figure 2.

3. The process of claims 1-2, wherein the monooxygenase is supplied with necessary reduction equivalents by a reduced metal complex especially comprising Cyclopentadienyl bipyridyl rhodium complexes wherein one or both of the pyridine rings can be substituted.

4. The process of claim 3, wherein the monooxygenase component is regenerated directly (Figure 1 and/or Figure 4) or at any point of its electron transport chain (e.g. replacing the NAD(P)H:acceptor oxidoreductase).

5. The process of claims 3-4, wherein the reduced complex is *in situ* regenerated either electrochemically or chemically.

6. The process of claims 3-4, wherein the reduction equivalents can be derived chemically either from formate or alcohols.

7. The process of claims 3-4, wherein the electrochemical supply with reduction equivalents is achieved from a cathode with a electrochemical potential in the range of -450 to -900 mV (vs. Ag/AgClsat.).

8. The process of claim 7, wherein the enzyme is separated from the electrochemical cell via compartmentation (Figure 6).

9. The process of claim 8, wherein the monooxygenase is retained from the electrochemical cell either by immobilization to a solid matrix within a plugged-flow-reactor or within a continuously stirred tank reactor or in an enzyme-membrane reactor.

10. The process of claim 7-9, wherein the oxygen supply for the biocatalyst is controllable so that at the efflux of the biocatalyst compartment the oxygen saturation is minimized.

11. The process of any one of claims 1-10 wherein a second phase, either solid or liquid is used during the reaction to extract any product as soon as it is formed to prevent product decay on in order to act as substrate reservoir.

12. The process of claim 11, wherein to phase contact is established either by direct contact (e.g. emulsion) or with constant phase separation (e.g. by a hollow-fiber module).

13. The process of any of claims 1-12, wherein inactivation of either biocatalyst or metal complex is prevented either by utilization of nucleophilic buffer additives (e.g. NH₃ or TRIS) or by immobilization of the metal complex.

14. The process of claims 3-12, wherein the monooxygenase can contain FAD, or FMN, or heme-iron or non-heme-iron or any other metal as cofactor or prosthetic group.

15. The process of claim 14, wherein the monooxygenase can be used for oxidation reactions such as:
a) oxidation of saturated, unsaturated aliphatic or aromatic carbon atoms, especially via hydroxylation, epoxidation or Baeyer-Villiger oxidation (insertion in C-C-bonds);
b) oxidation of heteroatoms from the groups III, V, VI, and VII within the substrate, especially oxidation of boron, nitrogen, phosporous, sulfur, selenium, bromine, and iodine.

16. A process for *in situ* generation of hydrogen peroxide coupled to an enzymatic reaction, preferably to a monooxygenase or a peroxidase for the oxidation of organic compounds.

17. The process of claim 16, wherein the reduced metal complex acts on alloxazine moieties such as FAD or FMN in the presence of molecular oxygen to form hydrogen peroxide.

18. The process of claims 16-17, wherein the reaction conditions are according to claims 4-17.

19. A process of *in situ* regeneration of NAD(P)⁺ from NAD(P)H.

20. The process of claim 19, wherein the oxidized coenzyme is consumed as cosubstrate by a dehydrogenase within an oxidation reaction.

21. The process of claims 19-20, wherein a inorganic mediator such as [Cp*Rh(bpy)(H₂O)]²⁺ is used to catalyze the transhydrogenation reaction from NAD(P)H to an alloxazine based acceptor (such as FAD, FMN).

22. The process of claims 19-21, wherein the reduced acceptor is reoxidized either by molecular oxygen of by anodic oxidation.

23. The process of claim 22, wherein the accumulation of hydrogen peroxide is prevented either by chemical or enzymatic dismutation.
